# FASCICULE DE BREVET EUROPEEN

(11) **EP 0 703 954 B1**
(45) Date de publication et mention de la délivrance du brevet: **19.12.2001**
(21) Numéro de dépôt: 94919709.9
(22) Date de dépôt: 14.06.1994
(51) Int. Cl.: C09K 15/08, C07D 311/62, C08H 5/00, A61K 31/352

(54) **COMPOSITIONS DE DERIVES DE POLYPHENOLS ET LEUR PREPARATION**
POLYPHENOLDERIVATZUSAMMENSETZUNGEN UND DEREN HERSTELLUNG
POLYPHENOL DERIVATIVE COMPOSITIONS AND PREPARATION THEREOF

(30) Priorité: 14.06.1993 FR 9307140
(43) Date de publication de la demande: 03.04.1996
(73) Titulaire: BERKEM, 24680 Gardonne (FR)
(72) Inventeur: VERCAUTEREN, Joseph, F-33600 Pessac (FR); WEBER, Jean-Frédéric, F-33000 Bordeaux (FR); BISSON, Jean-Louis, F-33000 Bordeaux (FR); BIGNON, Jean, F-33000 Bordeaux (FR)
(74) Mandataire: Peuscet, Jacques
(86) Numéro de dépôt international: FR9400712
(87) Numéro de publication internationale: WO9429404

(56) Documents cités:
- EP-A- 0 003 274
- EP-A- 0 039 844
- DE-A- 1 518 003
- FR-A- 2 345 441
- DATABASE WPI Week 8943, Derwent Publications Ltd., London, GB; AN 89-314140 & JP,A,1 233 277 (MITSUI NORIN KK) 19 Septembre 1989
- DATABASE WPI Week 9234, Derwent Publications Ltd., London, GB; AN 92-280098 & JP,A,4 190 774 (KIKKOMAN CORP) 9 Juillet 1992
- DATABASE WPI Week 8137, Derwent Publications Ltd., London, GB; AN 81-67122D & JP,A,56 095 182 (NORIINSHO) 1 Août 1981
- DATABASE WPI Week 8949, Derwent Publications Ltd., London, GB; AN 89-359900 & JP,A,1 268 683 (MITSUI NORIN KK)
- DATABASE WPI Week 9330, Derwent Publications Ltd., London, GB; AN 93-239909 & JP,A,5 163 131 (KANEBO)
- DATABASE WPI Week 9411, Derwent Publications Ltd., London, GB; AN 94-089260 & JP,A,6 040 883 (SUZUYO) 15 Février 1994
- DATABASE WPI Week 8810, Derwent Publications Ltd., London, GB; AN 88-067041 & JP,A,63 020 321 (HITACHI) 28 Janvier 1988
- CHEMICAL ABSTRACTS, vol. 94, no. 11, 16 Mars 1981, Columbus, Ohio, US; abstract no. 80196x, . 'dimeric procyanidins from juniperus sabina' page 379 ;colonne R ; & KHIM. PRIR SOEDIN, vol.3, 1980 page 420 PASHINA ET AL
- CHEMICAL ABSTRACTS, vol. 96, no. 26, 28 Juin 1982, Columbus, Ohio, US; abstract no. 223079r, OTSUKA HIROSHI 'studies on antiinflammatory agents. VI. antiinflammatory constituents of cinnamomum sisboldii meissn.' page 358 ; & YAKUGAKU ZASSHI, vol.30, no.2, 1982 pages 162 - 172

## Description

L'invention concerne de nouvelles compositions de dérivés de polyphénols et leur préparation.

Elle se rapporte plus particulièrement à des compositions renfermant des dérivés polyhydroxylés de flavane et spécialement de flavan-3-ol.

On rappelle que le noyau flavane répond à la structure (x) : 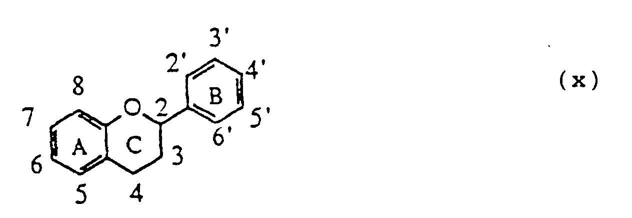 les flavan-3-ols possédant un groupe-OH en position 3.

Des flavanols, polyhydroxylés sur les noyaux benzéniques, peuvent être obtenus par extraction à partir de diverses sources végétales comme diverses espèces de pin, le thé vert ou la vigne. Les extraits bruts isolés sont formés de mélanges complexes comprenant des monomères et des polymères, plus particulièrement des oligomères allant des dimères et le plus généralement jusqu'aux décamères.

Les procédés d'extraction industriels visent à fournir des fractions constituées majoritairement d'oligomères. Ces fractions seront appelées indifféremment ci-après oligomères flavanoliques, ou encore oligomères procyanolidiques et en abrégé OPC.

Les groupes phénols présents sur les motifs flavanoliques confèrent à ces OPC des propriétés anti-radicalaires et anti-oxydantes qui présentent un intérêt potentiel pour de nombreuses applications.

Certains extraits d'OPC sont utilisés en thérapeutique comme protecteurs vasculaires ou encore en cosmétique.

L'utilisation pratique et plus large de ces produits se heurte toutefois au problème de leur instabilité due à la présence de groupements phénoliques libres.

Les phénols, de façon générale, sont des produits qui s'oxydent spontanément au contact de l'oxygène de l'air et/ou en présence de lumière, en faisant intervenir un mécanisme radicalaire que l'on peut représenter par l'équation suivante :

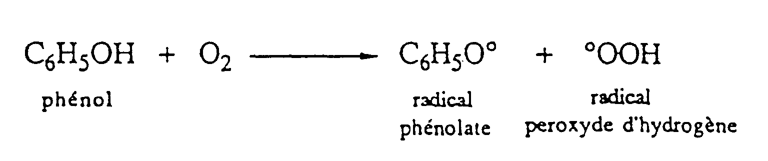

Le radical phénolate étant stabilisé par effet de résonance il se forme des dérivés radicalaires de type: 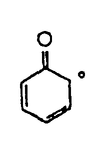 qui peuvent ensuite se coupler en ortho et para pour donner des produits de condensation des types suivants :

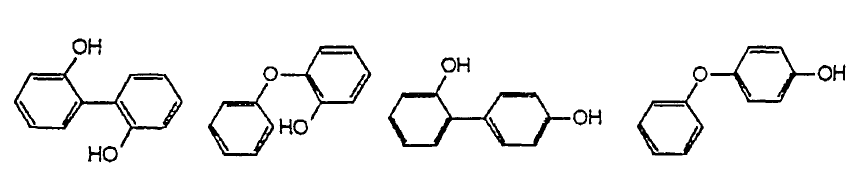

Les dérivés polyphénoliques qui suivent un tel mécanisme, donnent des produits de condensation radicalaire. A ces dérivés, lorsque la réaromatisation ne peut avoir lieu, s'ajoutent des produits de type quinonique. Cet ensemble de composés est responsable de l'apparition de colorations brun-rouge, incompatibles avec certaines applications.

De plus, les OPC sont des produits hydrosolubles, ce qui pose un problème de compatibilité avec bon nombre d'excipients utilisés généralement dans les applications mentionnées ci-dessus, qui présentent au contraire des propriétés liposolubles.

La recherche de moyens permettant de conférer une stabilité satisfaisante aux dérivés polyhydroxylés et en particulier aux OPC, et en même temps de les rendre liposolubles, a conduit les inventeurs à mettre au point une technique de protection des groupes -OH libres par estérification dans des conditions spécifiques.

L'invention a donc pour but de fournir des compositions de dérivés de polyphénols de grande stabilité.

Elle vise également à fournir un procédé d'estérification des fonctions phénol de ces compositions, de mise en oeuvre aisée, et exploitable à l'échelle industrielle.

L'invention vise en outre la mise à profit des propriétés anti-radicalaires et anti-oxydantes de ces compositions dans divers domaines, notamment en thérapeutique, en cosmétique et en diététique.

Les compositions de l'invention sont caractérisées en ce qu'elles comprennent des oligomères ou des polymères dont les motifs monomères répondent à la formule (I) : 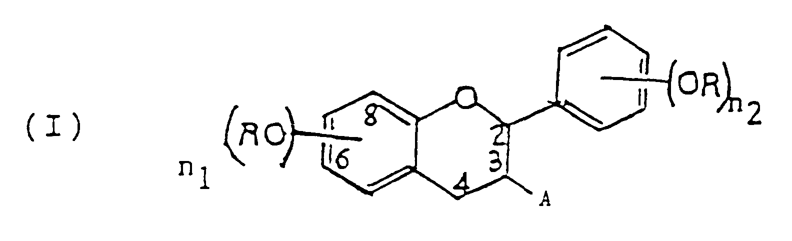 dans laquelle
- A représente un groupe -OR, un atome d'hydrogène, ou un substituant R,
- au moins la majorité des substituants R représente un groupe -COR1, R1 étant un radical alkyle d'au moins deux atomes de carbone, linéaire ou ramifié, saturé ou insaturé, un radical aryle, aralkyle ou aralkylène,
- le ou les autres substituants R qui ne représentent pas un groupe -COR1 étant un atome d'hydrogène, un groupe alkyle, un groupe acyle -CO-C₆H₂-(OH)₃, un ose ou un polyose, et
- n₁ et n₂, identiques ou différents l'un de l'autre, sont des nombres de 1 à 3, correspondant au nombre de substitutions sur un cycle,
et les diastéréoisomères et les régioisomères de ces motifs,
les motifs monomères étant reliés par des liaisons carbone-carbone ou par des ponts éthers entre les cycles constitutifs du noyau flavane.

Les compositions ainsi estérifiées sont d'une grande stabilité. Elles peuvent être conservées pendant au moins 2 ans dans des conditions normales de conservation (température de 10 à 22°C, en conditionnement protégeant de la lumière, hygrométrie 40-50 %).

Les oligomères ou les polymères de ces esters répondent plus spécialement à la formule II : 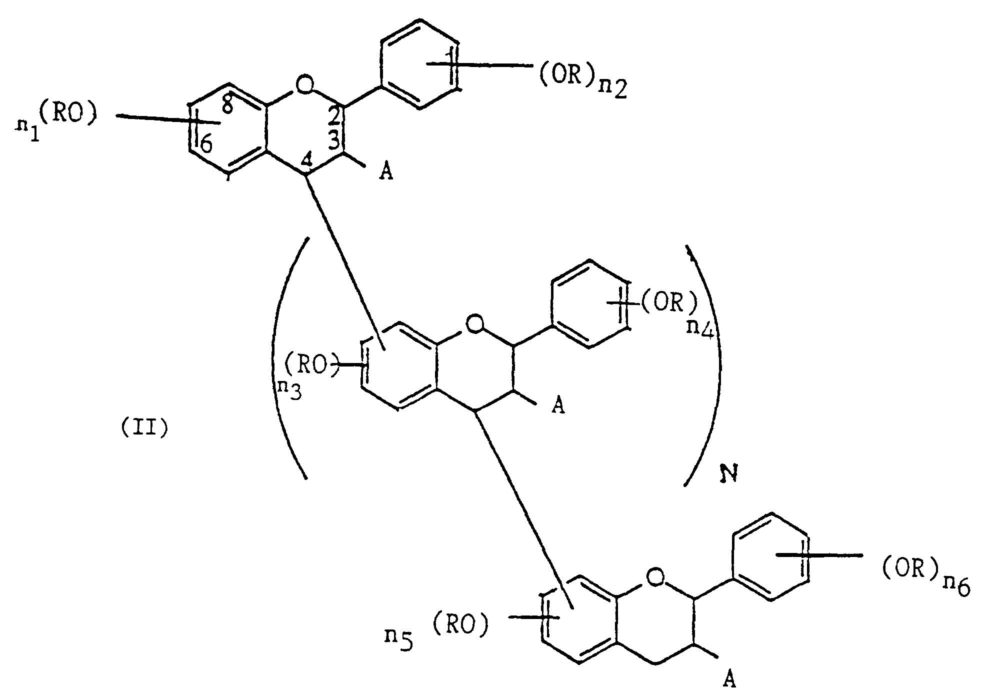 dans laquelle
- A et R sont tels que définis ci-dessus,
- n₁ à n₆, identiques ou différents les uns des autres, sont des nombres de 1 à 3, représentant le nombre de substituants sur un cycle benzénique, et
- N est un nombre de 0 à 100,
et les diastéréoisomères et régioisomères correspondants.

Dans une famille de l'invention, les liaisons entre les atomes de carbone des motifs successifs sont situées entre le C-4 d'un motif et le C-6 ou le C-8 d'un autre motif.

Une autre famille comprend en plus au moins deux motifs reliés par un pont oxygène. Des produits correspondants répondent à la formule III : 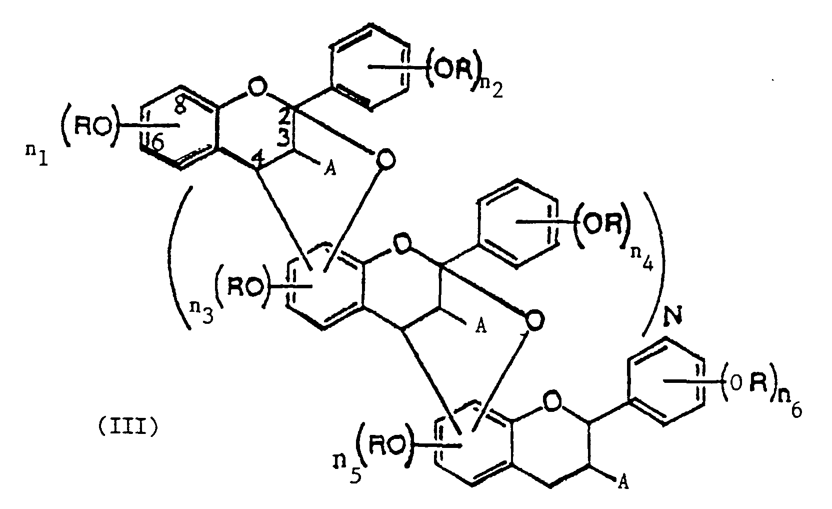 dans laquelle A, R, n₁ à n₆, et N sont tels que définis ci-dessus.

Les motifs monomères sont plus spécialement reliés par un pont éther entre le C-2 d'un motif et l'un des carbones C5 à C8 du motif suivant.

Dans un groupe de compositions de l'invention, N est égal à O dans la formule II ou III ci-dessus, les esters correspondant à des dimères.

Dans un autre groupe, N est un nombre de 1 à 10.

Dans encore un autre groupe, N est supérieur à 10, en particulier de 11 à 100.

Dans les compositions définies ci-dessus, R1 représente avantageusement un radical d'acide gras saturé ou insaturé, avec dans ce dernier cas des doubles liaisons cis, ce qui correspond au cas le plus fréquent chez les produits naturels, ou bien des liaisons trans, pour des produits obtenus plus particulièrement par synthèse ou hémisynthèse.

Des exemples d'acides gras sont donnés ci-après. Selon la nomenclature classique, on indique pour chacun d'eux le nombre d'atomes de carbone C, puis le nombre de doubles liaisons, et l'emplacement de ces doubles liaisons. Les noms des acides gras sont précisés pour les plus classiques.
Il s'agit des radicaux des acides butyrique C4:0 ; valérique C5:0 ; hexanoique C6:0, sorbique C6:2(n-2) ; C8:0 ; C11:1 ; C11:2 ; laurique C12:0 ; C13:0 ; C13:2 ; C14:0 ; C15:0 ; C15:2 palmitique C16:0 ; C16:1(n-7) ; C16:2(n-4) ; C16:2(n-7) ; C16:3(n-4) ; C16:4 ; C17:0 ; stéarique C18:0 ; oléïque C18:1(n-9) ; C18:1(n-7), linoléique C18:2(n-6) ; linolénique C18:3(n-6) ; α linolénique C18:3(n-3) ; C18:4(n-3) ;C20:0 ; C20:1(n-9) ; C20:2(n-6) ; C20:3(n-6) : C20:4(n-6) ; arachidonique C20:4(n-3) ; éicosapentaénoïque C20:5(n-3) ; C22:0 ; C22:1 ; C22:1(n-5) ; C22:3(n-3) ; C22:4 (n-6) ; C22:4(n-3) ; C22:5(n-3) ; C22:5(n-6) docosahexaénoïque C22:6(n-3) et C24:1(n-9).

Les radicaux des acides gras en C16 et plus sont particulièrement préférés en vue d'applications en cosmétique. Ces acides gras sont avantageusement extraits de microalgues.

Selon une autre disposition de l'invention R1 représente un groupe aryle tel que le radical phényle.

Selon encore une autre disposition, R1 représente un groupe aralkyle ou aralkylène, le groupe alkyle ou alkylène étant plus particulièrement en C1 à C8, notamment en C1 à C4. A titre d'exemples de groupes aralkyle et aralkylène, on citera le groupe benzyle et styryle.

Les compositions définies dans les diverses dispositions qui précèdent renferment en mélange avec les esters oligomères et/ou les esters polymères, qui constituent les produits principaux, des esters monomères.

L'invention vise en particulier les compositions de dérivés de flavanols. Dans ces dérivés le substituant A représente un groupe -OR, R étant tel que défini ci-dessus.

Il s'agit de manière préférée d'esters de dérivés de flavanols appartenant à la série catéchique.

Dans ces esters, les groupes oxygénés sont généralement au nombre de 5 par motif flavanolique, et occupent les positions 3, 5, 7, 3' et 4'.

Il sera fait référence dans les exemples aux peresters pour désigner les produits dans lesquels toutes les fonctions -OH sont estérifiées. Ces esters comportent le cas échéant un pont oxygène entre le C-2 et l'un des carbones C₅ à C₈.

Des esters de dérivés flavanoliques particulièrement préférés sont obtenus à partir des OPC extraits de sources végétales.

Les matières végétales les plus couramment utilisées comprennent diverses espèces de pin, la vigne et le thé vert.

Conformément à l'invention, les compositions définies ci-dessus sont obtenues en faisant réagir des compositions phénoliques correspondantes ayant au moins un groupe -OH libre avec un agent d'acylation susceptible de fournir le radical -COR1, R1 étant tel que défini ci-dessus, dans des conditions permettant la substitution d'au moins un groupe -OH libre par un radical acyle -COR1.

L'agent d'acylation est choisi avantageusement parmi les acides R₁COOH ou les dérivés de tels acides, en particulier les chlorures d'acides R1COCl, les anhydrides R1COOR1 ou les esters R1COOR₂, R₂ représentant un radical alkyle en C1-C8 ou aryle.

Lorsqu'on utilise l'acide comme agent d'acylation, on réalise avantageusement la réaction en présence d'un agent d'activation de ce dernier.

De manière la plus classique, cet agent est constitué par le dicyclohexylcarbodiimide, mais d'autres agents conférant le même effet d'activation peuvent être utilisés comme le ter-butylchloroformiate (pour formation d'un anhydride mixte).

La réaction d'acylation est effectuée en présence d'un solvant permettant une solubilisation partielle des composés polyphénoliques de départ.

Des solvants appropriés sont choisis parmi des dérivés halogénés comme le dichlorométhane, le chloroforme, 1,2-dichloroéthane ou une amine comme la pyridine.

La réaction est réalisée de préférence à la température ambiante.

La réaction avec le chlorure ou l'anhydride d'acide peut être réalisée en variante en milieu aqueux alcalin selon la réaction de Schotten Baumann.

On met alors en présence les dérivés polyphénoliques en phase aqueuse à un pH de 7,5 à 12, notamment de 8 à 10, l'agent acylant dissous en phase organique et un agent de transfert de phase.

La phase organique dans laquelle est dissous l'agent acylant est avantageusement un solvant organochloré tel que le chloroforme ou le dichlorométhane.

Comme agents de transfert de phase appropriés, on citera des halogénures ou des hydroxydes comme ceux de tétrabutylammonium ou de tétrabutyl phosphonium, les hydrogénosulfates par exemple de tétrabutylammonium, ou encore le chlorure de benzyltriéthylammonium.

Les dérivés acylés obtenus sont séparés du mélange réactionnel et purifiés en vue des applications envisagées. Des techniques appropriées comprennent l'extraction liquide-liquide, la chromatographie et/ou la précipitation.

Les compositions polyhydroxylées de départ sont avantageusement des produits du commerce. Dans le cas d'OPC, ces produits sont obtenus par extraction à partir de plantes. Il s'agit de préférence de fractions purifiées. Un procédé d'extraction classique dérive de celui décrit dans le brevet FR 1 427 100 (PV N° 998 508) du 14 décembre 1964. Les OPC sont extraits à partir de la matière végétale par une solution aqueuse saturée en NaCl. Une extraction liquide-liquide par de l'acétate d'éthyle est ensuite effectuée, puis on précipite les OPC en ajoutant du chloroforme en excès. Après filtration, le précipité est repris par de l'acétate d'éthyle et soumis le cas échéant, aux fins de purification supplémentaire, à plusieurs cycles de reprécipitation par du chloroforme en excès, reprise par de l'acétate d'éthyle, le solvant étant évaporé à la fin.

En variante, ces compositions sont extraites à partir de la matière végétale par de l'eau, puis on ajoute du NaCl. Les impuretés sont précipitées, éliminées par filtration et on procède à une extraction liquide-liquide des OPC à l'aide d'acétate d'éthyle. Le solvant est évaporé et le résidu repris par de l'eau. Après les étapes de lavage de la solution aqueuse par du chloroforme, séchage par atomisation ou reprise par de l'acétate d'éthyle, on précipite les compositions flavanoliques en ajoutant du chloroforme en excès, puis on filtre et on sèche à l'étuve.

Selon les techniques d'extraction utilisées, les OPC comportent un motif sucre comme indiqué plus haut. Il s'agit d'un ose comme par exemple le glucose ou le galactose, ou de polyoses formés de plusieurs de ces motifs oses, identiques ou différents. L'extraction de ces dérivés glycosylés est effectuée à partir de la matière végétale par de l'eau, un alcool tel que l'éthanol ou le méthanol, ou un mélange eau-acétone (2/3). Après lavage par de l'acétate d'éthyle, l'extrait est remis en solution aqueuse. On effectue une extraction liquide-liquide par du n-butanol, puis on élimine le solvant par évaporation. Le résidu est soumis à une purification chromatographique à contre-courant ou sur Fractogel TSK HW40, Séphadex LH20, gel MCI CHP 20P, gel de silice RP C18.

Les travaux effectués sur les compositions de dérivés de polyphénols de l'invention ont montré que la présence des groupes esters protecteurs permet de favoriser le transport de ces compositions au travers des membranes biologiques et d'atteindre localement des concentrations plus importantes qu'avec des composés non acylés, selon le concept de prodrogue.

Au contact d'estérases, présentes dans la plupart des milieux et tissus biologiques, au moins une partie des groupements protecteurs ester sont éliminés en régénérant les composés phénoliques de départ et les acides R1COOH. Ces composés exercent en combinaison leurs propres activités et ce, avantageusement, d'une manière synergique.

Ces propriétés avantageuses s'accompagnent en outre d'une grande innocuité comme démontré pour les compositions polyphénoliques natives et notamment pour les compositions flavanoliques, ainsi que pour les acides R1COOH.

Ces compositions sont donc particulièrement appropriées pour l'élaboration de préparations pharmaceutiques.

Les préparations pharmaceutiques de l'invention renferment une quantité efficace d'au moins une composition phénolique telle que définie ci-dessus, en particulier d'une composition flavanolique, en association avec un véhicule pharmaceutique inerte.

Des préparations pharmaceutiques avantageuses renferment ces dérivés seuls ou en association avec des médicaments à effet protecteur vis-à-vis des réactions d'oxydation. A titre d'exemple, on citera le β-carotène ou la vitamine E.

Compte tenu de leurs propriétés anti-radicalaires et anti-oxydantes, ces préparations pharmaceutiques sont utilisables notamment dans les indications thérapeutiques suivantes : troubles circulatoires, insuffisances veinolymphatiques, fragilité capillaire cutanée, troubles impliquant la circulation rétinienne, crise hémoroïdaire, érythèmes solaires ou liés à l'action de radiations, par exemple dans le cas de radiothérapies.

Les préparations pharmaceutiques de l'invention sont administrables par voie orale.

On a recours en particulier à des comprimés, pilules, tablettes, gélules, ou encore à des gouttes. Ces préparations renferment avantageusement de 50 à 200 mg d'équivalent OPC par unité de prise, de préférence de 100 à 150 mg.

On peut également administrer les compositions de l'invention par voie transdermique à l'aide de patchs ou encore sous forme de spray nasal.

En radiothérapie, ces compositions peuvent être utilisées en prévention des muccites, sous forme de gel appliqué directement sur les muqueuses succeptibles d'être irradiées.

A titre indicatif, la posologie utilisable chez l'homme, pour les pathologies considérées, correspond aux doses suivantes : phlébologie et lymphologie, 100 à 600 mg par jour en deux prises ; ophtalmologie, 100 à 400 mg par jour ; crise hémorroïdaire aigüe : 200 mg à 1, 2 g par jour, radiothérapie : pâte à 1-5 % d'équivalent de polyphénol non estérifié.

Les propriétés anti-radicalaires et anti-oxydantes de ces compositions sont également avantageusement mises à profit pour l'élaboration de préparations cosmétiques.

Dans ces préparations, les compositions sont associées à des véhicules appropriés pour un usage externe. On notera que leur caractère liposoluble favorise leur incorporation dans les formes galéniques habituellement utilisées en cosmétique.

Pour ces applications, les préparations se présentent sous forme de crème, pommade, émulsion, gel, liposomes, lotion. Elles renferment de 0,5 à 5 % de produit actif.

Selon un autre aspect de grand intérêt, les compositions de l'invention sont utilisables en diététique. Grâce notamment à leurs propriétés anti-radicalaires, elles assurent une meilleure conservation des aliments. De plus, elles constituent généralement un apport de facteur vitaminique, notamment de vitamine P avec les compositions flavanoliques. Elles sont donc ajoutées avec avantage aux boissons, par exemple aux jus de fruits, boissons toniques, aux produits laitiers et dérivés comme le beurre.

Elles sont également utilisables telles quelles sous forme liquide, ou encore en granulés ou analogues, gels ou sous forme de pâte, par exemple incorporées dans des confiseries comme les pâtes de fruits, bonbons, pâtes à mâcher.

Dans ces formes d'application, les compositions de l'invention peuvent être mélangées à des produits d'intérêt comme des vitamines et/ou des oligoéléments et/ou des huiles de poisson riches en acides gras insaturés, comme les huiles de flétan, de maquereau ou de saumon.

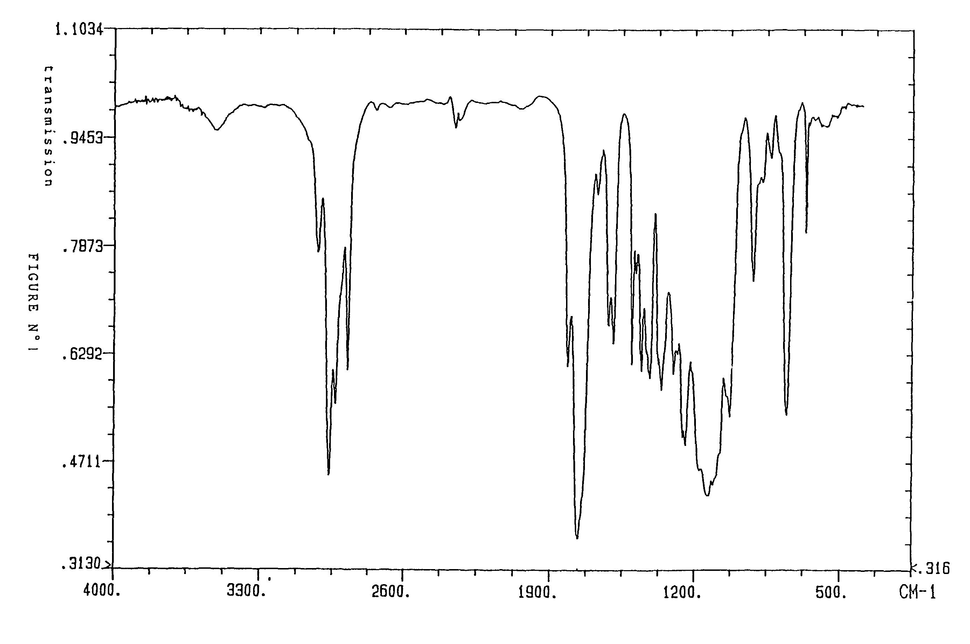

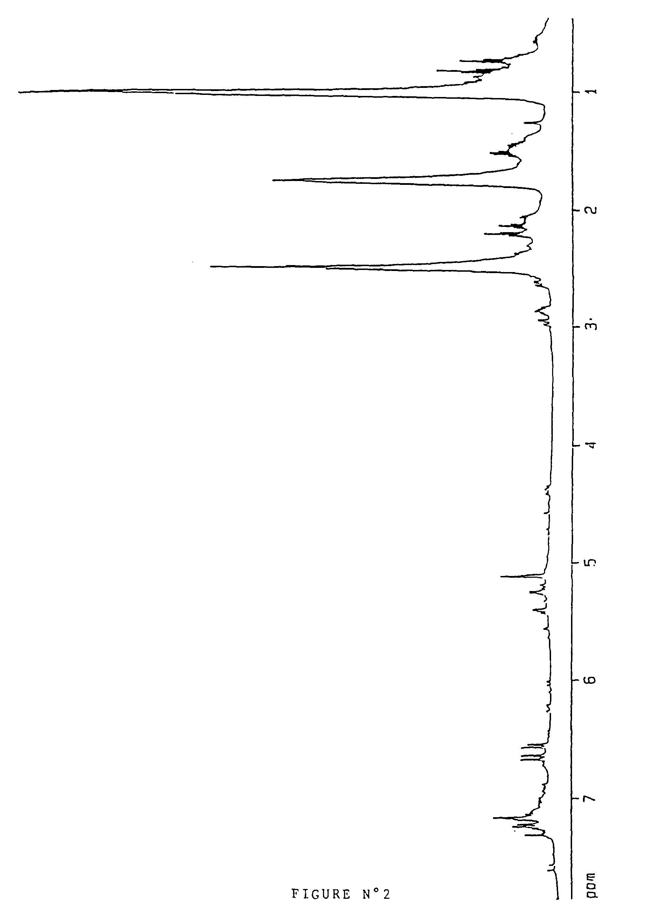

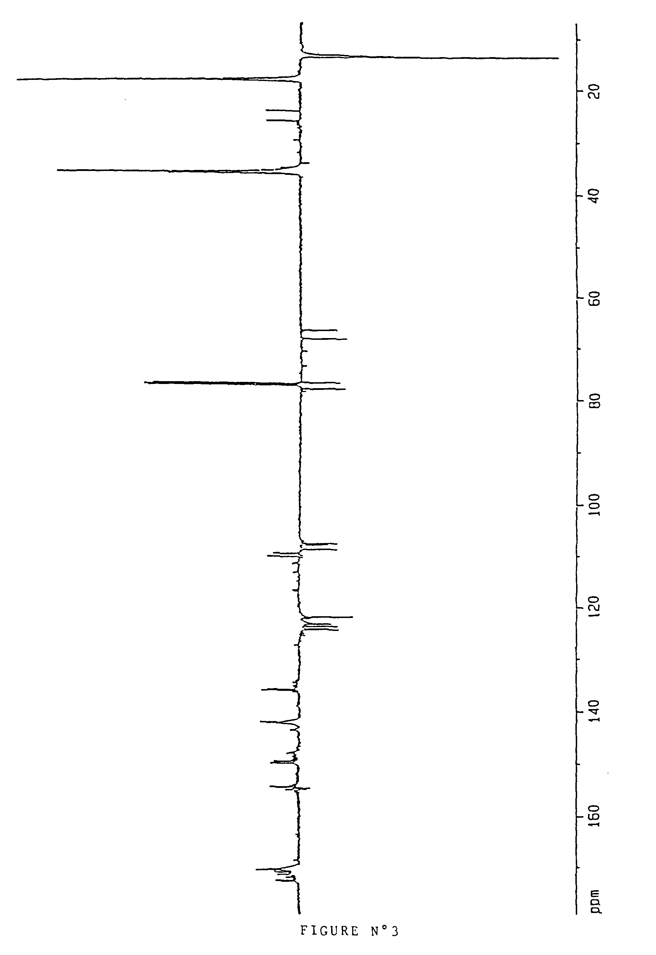

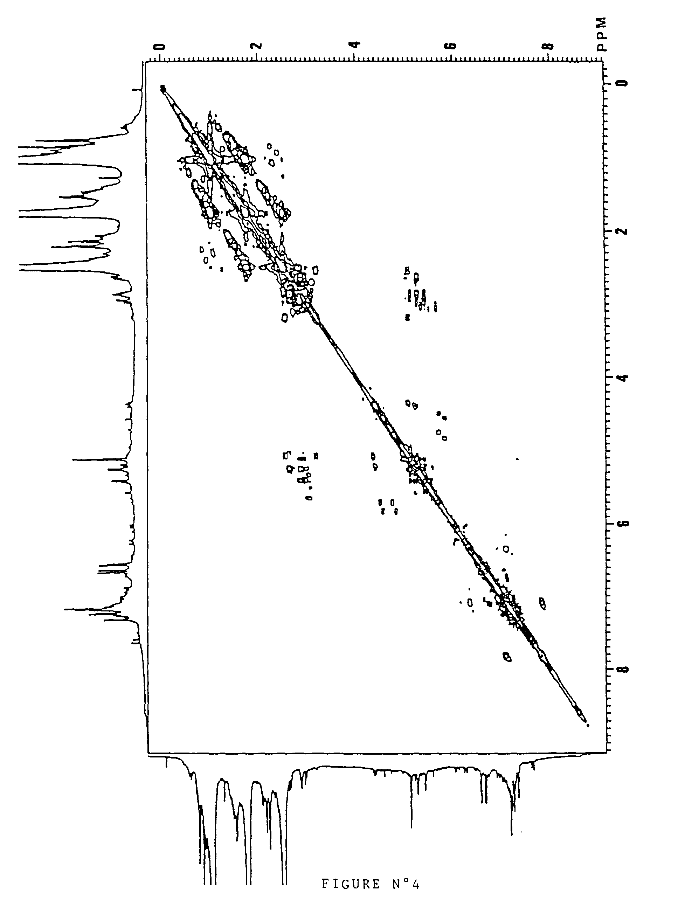

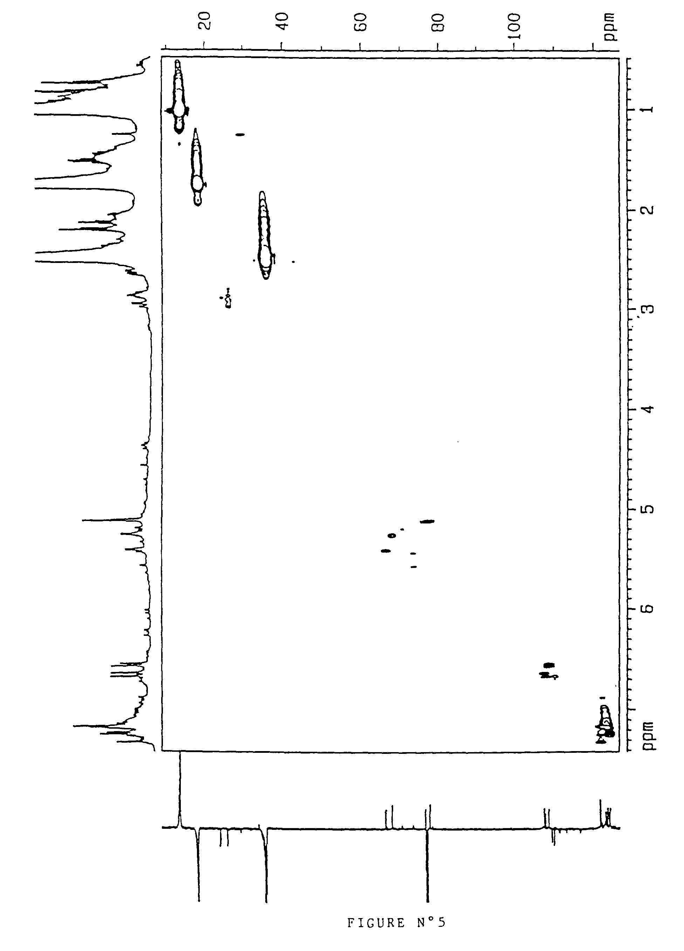

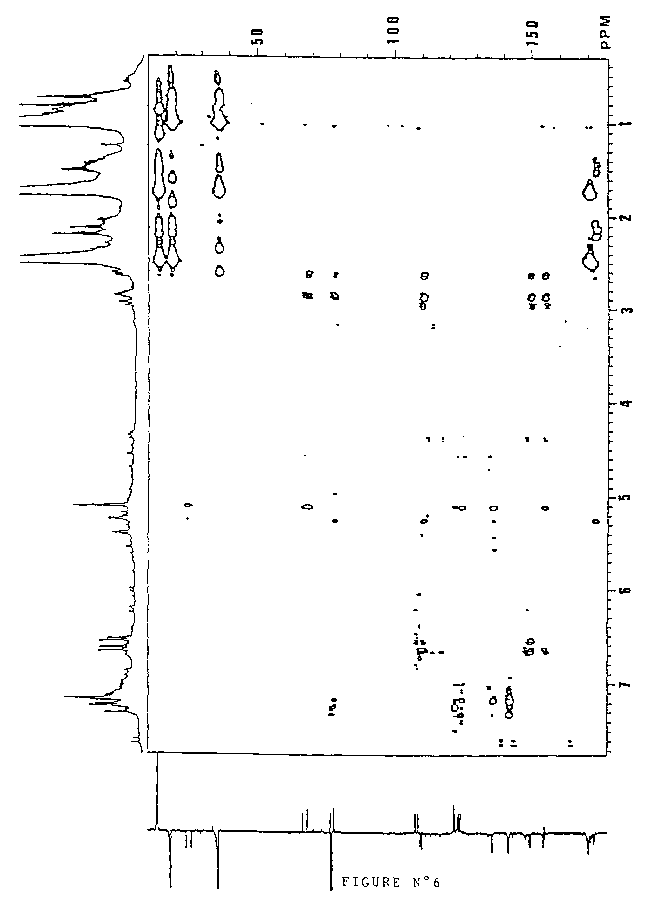

D'autres caractéristiques et avantages de l'invention apparaîtront dans les exemples qui suivent relatifs à la préparation de peresters d'OPC et à leur utilisation pour l'élaboration de médicaments ou de préparations cosmétiques. Dans ces exemples, il est fait référence aux figures 1 à 13 qui représentent respectivement :
- les figures 1 à 6 les spectres UV, IR et RMN 1^{H} et ¹³C mono et bidimensionels du perbutyrate d'OPC de vigne,
- les figures 7 et 8 les spectres IR et RMN ¹H du perlaurate d'OPC de vigne,
- la figure 9 le spectre IR du perpalmitate d'OPC de vigne,
- la figure 10 le spectre IR du perlaurate d'OPC de pin maritime,
- les figures 11 et 12 les spectres IR et RMN ¹H du perstéarate d'OPC de thé vert,
- la figure 13 le spectre IR du peroléate d'OPC de vigne.

### Exemple 1 : Préparation de perbutyrate d'OPC de vigne.

On utilise une fraction d'OPC telle qu'obtenue à partir de la vigne selon la méthode décrite dans le brevet FR 1 427 100 (N° PV 998 508) mentionné plus haut.

A 10 g de cette fraction, dissous dans 200 ml de pyridine et maintenus sous agitation, on ajoute goutte à goutte 20 ml de chlorure de butyryle. On laisse sous agitation, à 70°C, à l'abri de l'air (sous léger flux d'azote) et de la lumière durant 3 heures. Après concentration sous pression réduite, le résidu est repris par 200 ml de chloroforme, puis cette phase organique est lavée avec deux fois 250 ml d'une solution 0,1 N d'HCl, deux fois 200 ml d'eau distillée, deux fois 250 ml d'une solution 0,1 M de Na₂CO₃, puis trois fois 200 ml d'eau distillée. La phase chloroformique est récupérée, séchée sur Na₂SO₄ anhydre, filtrée, puis le solvant est évaporé sous pression réduite. Le produit ainsi obtenu est contrôlé par spectrométrie. Les spectres IR, RMN ¹H et ¹³C mono- et bidimensionnelle COSY, HMBC, HMMBC représentés sont représentés respectivement sur les figures 1 à 6.

### Exemple 2 : Préparation de pervalérate d'OPC de thé vert.

On utilise une fraction d'OPC telle qu'obtenue à partir du thé vert selon la méthode décrite dans le brevet FR 1 427 100 mentionné plus haut.

A 1 g de cette fraction, dissous dans 50 ml de pyridine et maintenu sous agitation, on ajoute goutte à goutte 3,5 ml d'anhydride valérique. On laisse sous agitation, à température ambiante, à l'abri de l'air (sous léger flux d'azote) et de la lumière durant 12 heures. Après concentration sous pression réduite, le résidu est repris par 50 ml de chloroforme, puis cette phase organique est lavée avec deux fois 50 ml d'une solution 0,1 N d'HCl, deux fois 50 ml d'eau distillée, deux fois 50 ml d'une solution 0,1 M de Na₂CO₃, puis trois fois 50 ml d'eau distillée. La phase chloroformique est récupérée, séchée sur Na₂SO₄ anhydre, filtrée, puis le solvant est évaporé sous pression réduite. Le produit ainsi obtenu est contôlé par spectrométrie.

### Exemple 3 : Préparation de perhexanoate d'OPC de vigne.

A 1 g d'OPC de vigne tels que mentionnés dans l'exemple 1, dissous dans 50 ml de pyridine et maintenu sous agitation, on ajoute goutte à goutte 4,8 ml de chlorure d'hexanoyle. On laisse sous agitation, à 60°C, à l'abri de l'air (sous léger flux d'azote) et de la lumière durant 5 heures. Après concentration sous pression réduite, le résidu est traité comme dans l'exemple 2. Le produit ainsi obtenu est contrôlé par spectrométrie.

### Exemple 4 : Préparation de perhexanoate d'OPC de thé vert.

A 1 g d'OPC de thé vert tels que mentionnés dans l'exemple 2, dissous dans 20 ml d'eau distillée, on ajoute 100 ml de dichloro-1,2-éthane et on soumet le mélange à une agitation vive (agitation mécanique à environ 1000 tours par minute). On ajoute 100 ml d'une solution aqueuse tamponnée à 0,1 M de phosphate de sodium et d'hydrogénophosphate de sodium (pH voisin de 12,3). On ajoute 100 mg de chlorure de tétrabutylphosphonium (agent de transfert de phase), puis 2,9 ml de chlorure d'hexanoyle. On laisse 45 minutes sous agitation vive. A la fin de la réaction, on récupère la phase organique et on la lave avec deux fois 100 ml d'une solution de soude 0,1 N, puis avec deux fois 100 ml d'eau distillée. On récupère la phase organique et on évapore sous pression réduite. Le produit ainsi obtenu est contrôlé par spectrométrie.

### Exemple 5 : Préparation de persorbate d'OPC de vigne.

A 1 g d'OPC de vigne tels que mentionnés dans l'exemple 1, dispersé dans 50 ml de dichloro-1, 2-éthane, sont ajoutés, sous agitation, 2,3 g d'acide sorbique, puis 4,3 g de dicyclohexylcarbodiimide (DCC). On laisse sous agitation, à température ambiante, à l'abri de l'air (sous léger flux d'azote) et de la lumière durant 2 heures. La phase organique est filtrée, puis le solvant est éliminé sous pression réduit. Le résidu est repris par 50 ml d'hexane. La solution hexanique est filtrée, puis lavée avec deux fois 100 ml d'une solution de soude 0,1 N, puis avec deux fois 100 ml d'eau distillée. On récupère la phase organique et on l'évapore sous pression réduite. Le produit ainsi obtenu est contrôlé par spectrométrie.

### Exemple 6 : Préparation de perlaurate d'OPC de vigne.

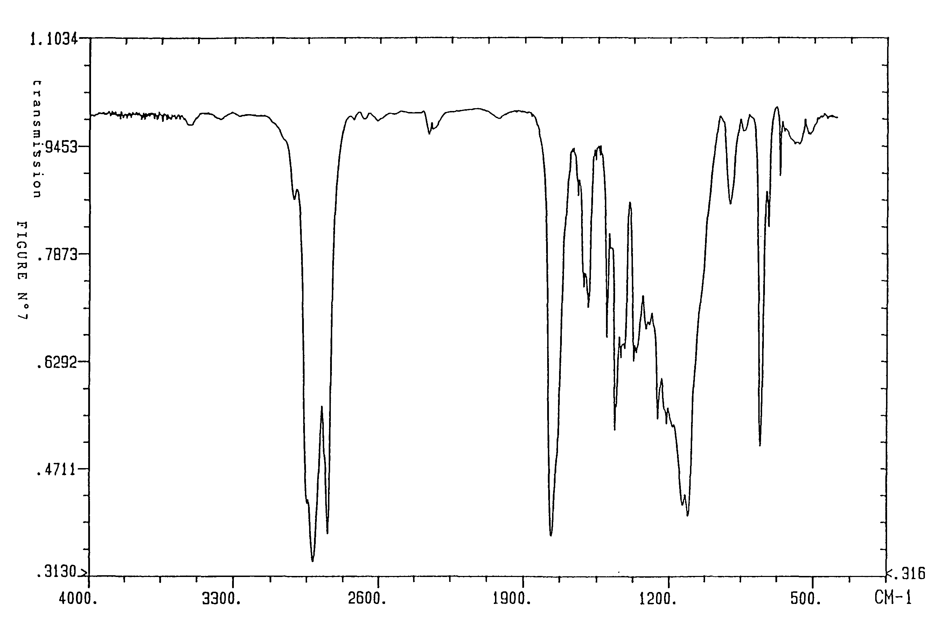

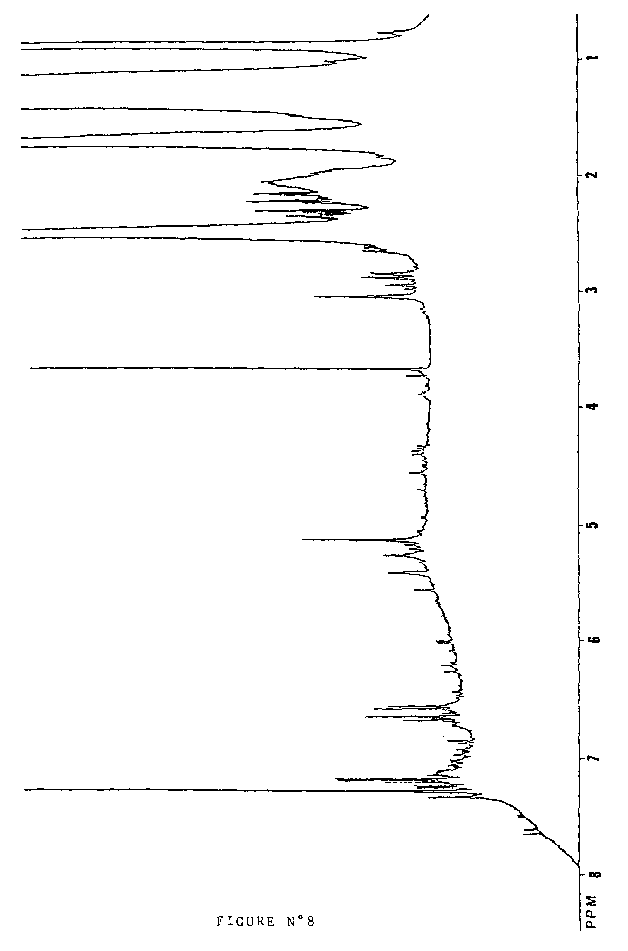

A 1 g d'OPC de vigne tels que mentionnés dans l'exemple 1, dispersé dans 50 ml de dichlorométhane, sont ajoutés, sous agitation, 4,2 g d'acide laurique, puis 4,3 g de dicyclohexylcarbodiimide (DCC). On laisse sous agitation, à température ambiante, à l'abri de l'air (sous léger flux d'azote) et de la lumière durant 2 heures. La solution réactive est traitée comme dans l'exemple 5. Le produit obtenu est contrôlé par spectrométrie (les spectres IR et RMN ¹H sont représentés sur les figures 7 et 8).

### Exemple 7 : Préparation de perlaurate d'OPC de pin maritime.

On utilise une fraction d'OPC telle qu'obtenue à partir du pin maritime selon la méthode décrite dans le brevet FR 998 508 mentionné plus haut.

A 1 g de cette fraction, dissous dans 50 ml de pyridine et maintenu sous agitation, on ajoute goutte à goutte 7,8 ml de chlorure de lauroyle. La réaction s'effectue selon les conditions décrites dans l'exemple 2. Le produit obtenu est contrôlé par spectrométrie.

### Exemple 8 : Préparation de perpalmitate d'OPC de vigne.

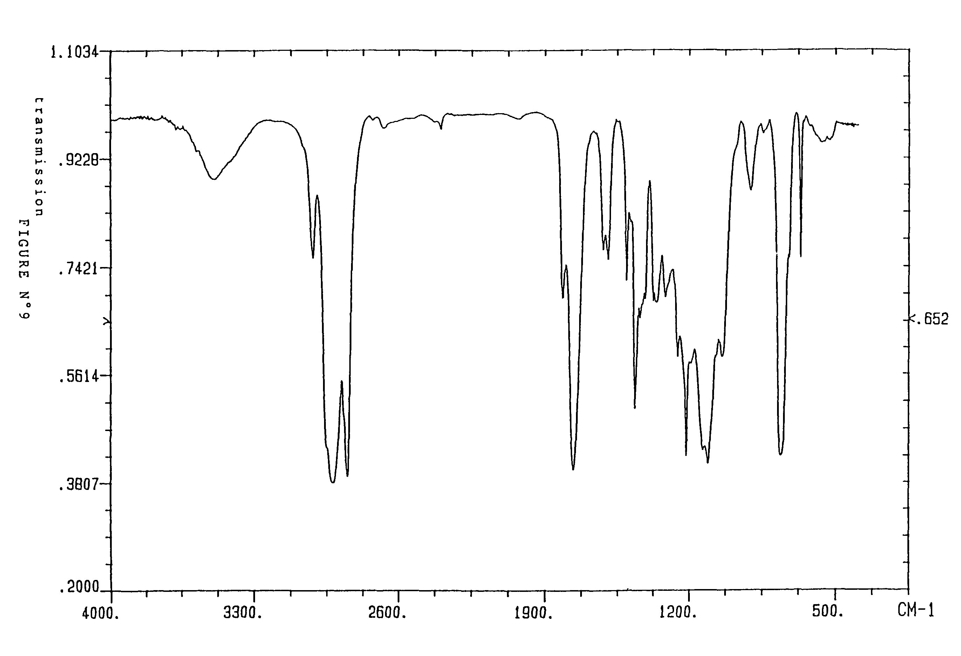

Dans un ballon d'un litre, on introduit sous azote 50 g d'OPC de vigne tels que mentionnés dans l'exemple 1 et 250 ml de pyridine. On agite jusqu'à dissolution totale. A l'aide d'une ampoule à addition, on ajoute lentement (environ 1 heure) 280 ml de chlorure de palmitoyle. La réaction est exothermique. On laisse le mélange sous agitation pendant 3 heures. Le mélange est alors revenu à température ambiante et a tendance à prendre en masse. On ajoute 250 ml de chloroforme et on maintient l'agitation à température ambiante pendant 12 heures. On évapore le solvant à sec sous pression réduite, puis on reprend le résidu par 1 l de chloroforme. On lave à deux reprises avec 500 ml d'acide chlorydrique 1 N, puis à deux reprises avec 500 ml d'eau distillée. On sèche sur 10 g de sulfate de calcium, on filtre, puis on évapore à sec sous pression réduite sans dépasser 40°C. Le résidu est repris par 500 ml d'acétone. Le mélange est soumis à agitation aux fins de dispersion (environ 1 heure). On filtre et on sèche ensuite en étuve ventilée à environ 25°C pendant 12 heures. On obtient environ 150 g de produit qui est contrôlé par spectrométrie (le spectre IR est représenté sur la figure 9).

### Exemple 9 : Préparation de perpalmitate d'OPC de pin maritime.

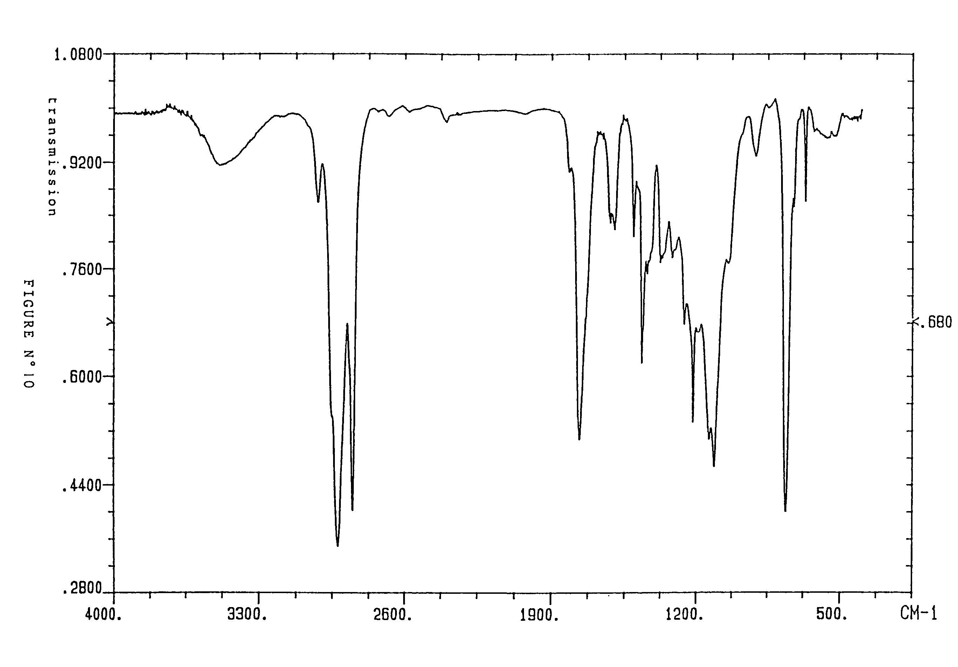

A 1 g d'OPC de pin maritime, tels que mentionnés dans l'exemple 7, dissous dans 20 ml d'eau distillée, on ajoute 100 ml de chloroforme puis on soumet le mélange à une agitation vive (agitation mécanique à environ 1000 tours par minute). On ajoute 100 ml d'une solution aqueuse tamponnée à 0,1 M de phosphate de sodium et d'hydrogénophosphate de sodium (pH de l'ordre de 12,3). On ajoute 120 mg d'hydrogénosulfate de tétrabutylammonium (agent de transfert de phase), puis 5,2 ml de chlorure de palmytoyle. On laisse 45 minutes sous agitation vive. A la fin de la réaction, on récupère la phase chloroformique et on la traite selon l'exemple 4. Le produit ainsi obtenu est contrôlé par spectrométrie (le spectre IR est représenté sur la figure 10).

### Exemple 10 : Préparation de perstéarate d'OPC de thé vert.

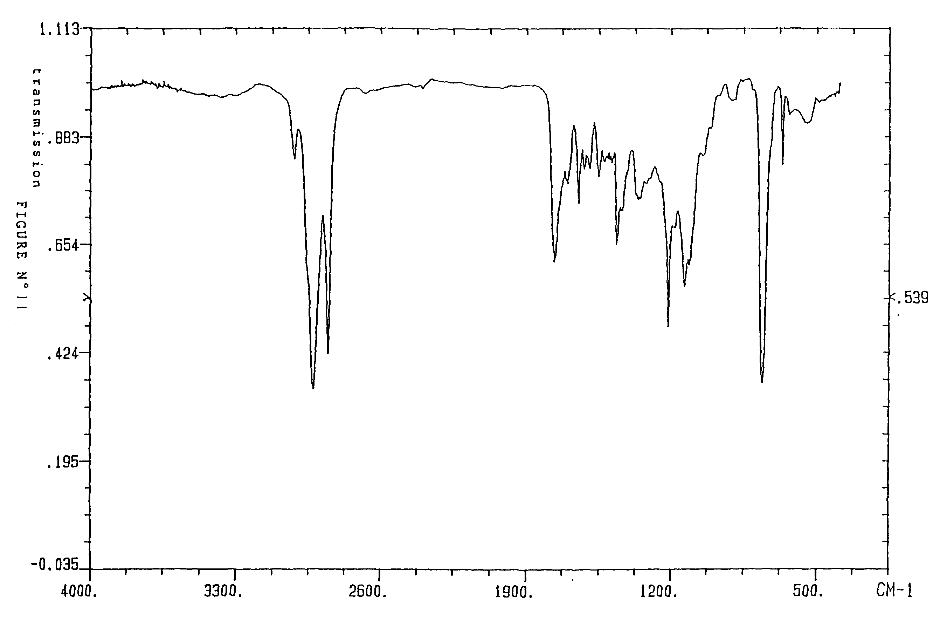

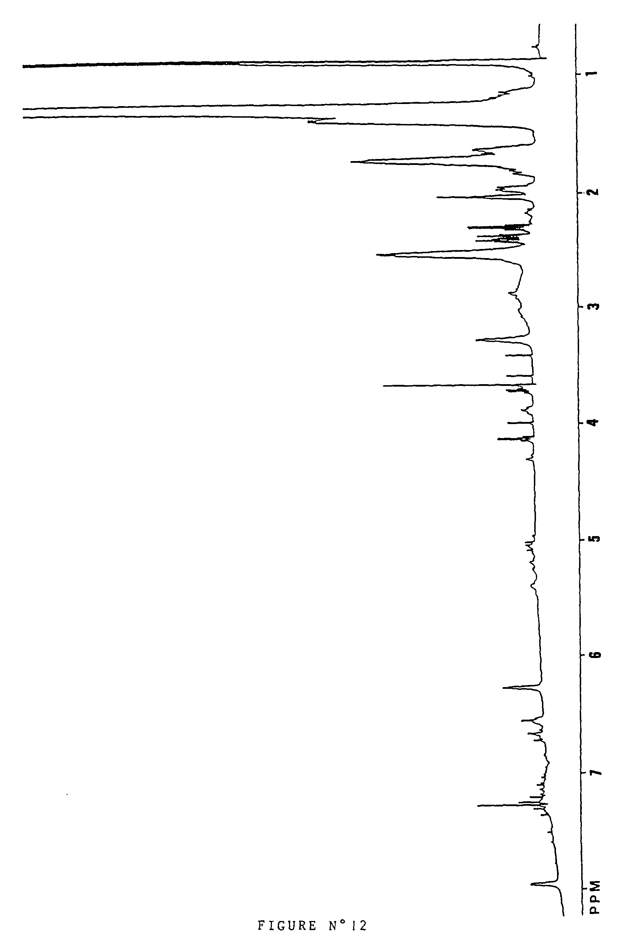

A 1 g d'OPC de thé vert tels que mentionnés dans l'exemple 2, sont ajoutés 5 g d'acide stéarique en solution dans 80 ml de dichloro-1,2-éthane. Le mélange est soumis à agitation. On dissout 3,6 g de dicyclohexylcarbodiimide (DCC) dans 20 ml de dichloro-1,2-éthane et on verse cette solution à la précédente. Le mélange est laissé sous agitation à température ambiante, à l'abri de l'air (sous léger flux d'azote) et de la lumière durant 15 heures. La solution réactive est traitée comme dans l'exemple 5. Le produit ainsi obtenu est contrôlé par spectrométrie (les spectres IR et RMN ¹H sont représentés sur les figures 11 et 12).

### Exemple 11 : Préparation de perstéarate d'OPC de vigne :

A 1 g d'OPC de vigne tels que mentionnés dans l'exemple 1 dissous dans 20 ml d'eau distillée, on ajoute 100 ml de dichlorométhane et on soumet le mélange à une agitation vive (agitation mécanique à environ 1000 tours par minute). On ajoute 100 ml d'une solution aqueuse tamponnée de phosphate de sodium et d'hydrogénophosphate de sodium (pH voisin de 11), 110 mg de chlorure de benzyltriéthylammonium, puis 6,0 ml de chlorure de stéaroyle (en une seule fois). On laisse 45 minutes sous agitation vive. A la fin de la réaction, on récupère la phase organique et on la traite selon le protocole décrit dans l'exemple 4. Le produit ainsi obtenu est contrôlé par spectrométrie.

### Exemple 12 : Variante de préparation de perstéarate d'OPC de vigne :

A 1 g d'une fraction oligoprocyanidolique (OPC) telle que mentionnée ci-dessus dissous dans 100 ml de pyridine et maintenu sous agitation, on ajoute goutte à goutte 10,4 g de chlorure de stéaroyle en solution dans 25 ml de pyridine. On laisse le mélange sous agitation, à température ambiante, à l'abri de l'air (sous léger flux d'azote) et de la lumière durant 12 heures. Après concentration sous pression réduite, le résidu est repris par 100 ml de chloroforme, puis cette phase organique est lavée avec deux fois 150 ml d'une solution 0,1 M d'HCl, deux fois 150 ml d'eau distillée, deux fois 150 ml d'une solution 0,1 M de Na₂CO₃ puis deux fois 150 ml d'eau distillée. La phase chloroformique est récupérée, séchée sur Na₂SO₄ anhydre, filtrée, puis le solvant est évaporé sous pression réduite. Le résidu ainsi obtenu est purifié par chromatographie préparative sur silice activée avec le système de solvant chloroforme-méthanol à 0, 5 %. Le produit ainsi obtenu est contrôlé par spectrométrie.

### Exemple 13 : Préparation de peroléate d'OPC de vigne.

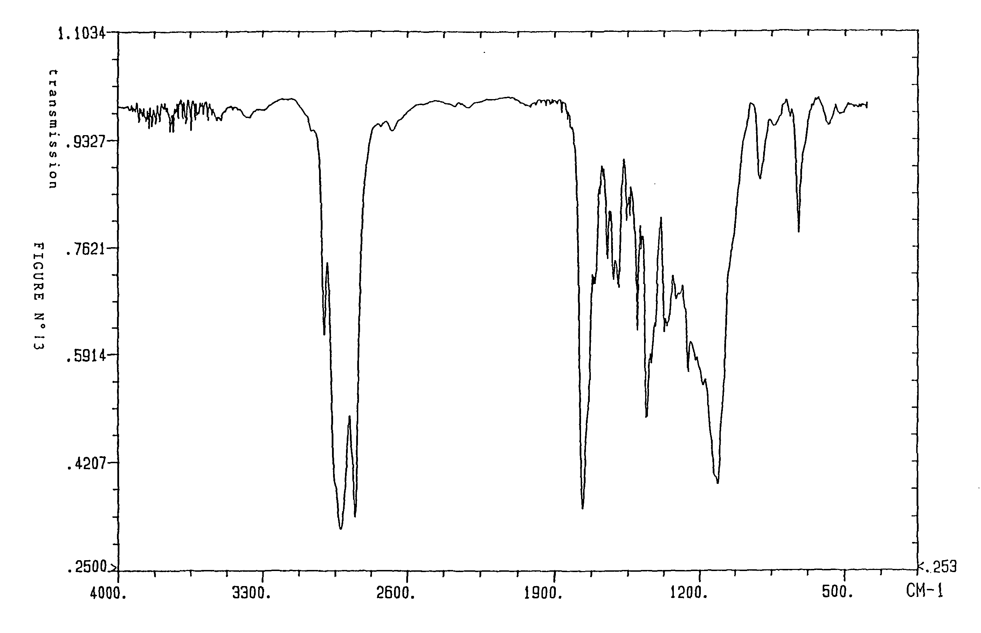

A 1 g d'OPC de vigne tels que mentionnés dans l'exemple 1 dispersés dans 50 ml de chloroforme, sont ajoutés, sous agitation, 6,5 ml d'acide oléique, puis 4,3 g de dicyclohexylcarbodiimide (DCC). Le mélange est laissé sous agitation, à température ambiante, à l'abri de l'air, (sous léger flux d'azote) et de la lumière durant 5 heures. La solution réactive est traitée comme dans l'exemple 10. Le produit obtenu est contrôlé par spectrométrie. Le spectre IR est représenté sur la figure 13.

### Exemple 14 : Préparation de stéarates de flavanols.

On utilise une fraction oligoprocyanidolique (OPC) telle qu'obtenue à partir de la vigne selon la méthode décrite dans l'exemple 1.

A 1 g de cette fraction, on ajoute 9 g d'acide stéarique, 7 g de dicyclohexylcarbodiimide (DCC) et 100 ml de chloroforme.

On porte le mélange au reflux sous agitation pendant 24 heures.

Le précipité de dicyclohexylurée formé est éliminé par filtration. La phase organique est évaporée, reprise par CHCl₃ et le résidu soumis à une purification chromatographique.

### Exemple 15 : Variante de préparation de stéarates de flavanols.

On utilise une fraction OPC telle qu'obtenue ci-dessus.

On fait réagir 1 g d'une telle fraction avec 17,5 g d'anhydride stéarique ou 9,5 g de chlorure de stéaroyle dans 100 ml de pyridine. On porte le mélange à 80°C sous agitation pendant 24 heures. On évapore la plus grande partie de la pyridine, puis on reprend la phase restante par CHCl₃. La pyridine restante est extraite à l'aide d'une solution aqueuse acide, et la composition obtenue est soumise à une purification par chromatographie.

### Exemple 16 : Préparation d'oléates de flavanols.

On procède comme dans l'exemple 15 mais en remplaçant l'anhydride stéarique et le chlorure de stéaroyle par le dérivé d'acide oléique correspondant.

### Exemple 17 : Préparation de sorbate de flavanols.

En procédant comme décrit dans l'exemple 15 mais en utilisant, comme dérivé d'acide, l'anhydride sorbique ou le chlorure de sorbyle, on obtient l'ester recherché, comme démontré par analyse RMN 2D.

### Exemple 18 : Préparation d'hexanoate de flavanols.

En opérant comme décrit dans l'exemple 15, mais en utilisant de l'anhydride hexanoïque ou du chlorure d'hexanoyle, on obtient l'hexanoate recherché.

### Exemple 19 : Préparation cosmétique antisolaire.

On réalise une émulsion antisolaire à propriétés antivieillissement cutané en mélangeant un filtre solaire avec un ester selon l'invention et des excipients pour crème.

Exemple de formulation :

| | |
|---|---|
| Néo Héliopan E 1000^{R} (isopropylméthoxycinnamate et éthyldiisopropylcinnamate) | 3 % |
| peroléate d'OPC selon l'exemple 13 | 3 % |
| excipients pour crème E/H | qs |

Composition d'excipients :
- propylène glycol dicaprylate/dicarate + stéaralkonium hectorite + propylène carbonate (Miglyol 840 gel B ^{R}) 20,0 %
- Bis-diglycéryl caprylate/caprate/isostéarate/ hydroxystéarate adipate (Softisan 649^{R} 5,0 %
- Isostéaryl diglycéryl succinate (Imwitor) 780 K^{R} 5,0 %
- Huile de paraffine 8,0 %
- Paraffine solide 3,0 %
- Sulfate de magnésium 2,0 %
- Eau qs 100 %

### Exemple 20 : Préparation cosmétique antiacnéique.

On prépare une crème H/E astringente et antiseptique pour peaux grasses en mélangeant du persorbate d'OPC selon l'exemple 5 à raison de 1 % avec un excipient pour crème H/E.

Comme formulation d'excipients, la composition suivante a été utilisée :
- Glycéryl stéarate SE (Imwitor 960^{R} en paillettes) 8,0 %
- Succinate caprylique/caprique/diglycéryle (Miglyol 829 ^{R}) 5,0 %
- Glycéryl ricinéolate (Softisan 701^{R}) 5,0 %
- Glycéryl laurate (Imwitor 312 ^{R}) 5,0 %
- Bis-diglycéryl caprylate/caprate/ isostéarate/hydroxystéarate adipate (Softisan 649 ^{R}) 3,0 %
- Huile de silicone 344 fluide 1,0 %
- Eau qs 100 %

### Exemple 21 : Préparation de médicament veinotonique et vasculoprotecteur.

On prépare des gélules à partir de 270 mg de perhexanoate d'OPC de vigne (correspondant à 100 mg d'OPC) selon l'exemple 3 et d'excipients pour un enrobage gastrorésistant comme l'acétophtalate de cellulose.

### Exemple 22 : Préparation de gélules pour utilisation en diététique.

On mélange du perlaurate d'OPC selon l'exemple 6 avec du sélénium et de la vitamine E ;
- perlaurate d'OPC : 105 mg (correspondant à 25 mg d'OPC)
- acétate de DL-α- tocophérol 40 mg
- sélénium : 50 mg

### Exemple 23 : gel buccal utilisable en radiothérapie.

On formule la composition suivante :
- gel de Carbopol R 934 P à 2 % 89,85 g
- para hydroxybenzoate de méthyle sodé 0,13 g
- para hydroxybenzoate de propyle sodé 0,02 g
- Labrafil ^{R} 5 g
- perhexanoate d'OPC de vigne selon l'exemple 3 5 g
(correspondant à 2 g d'OPC de vigne)

## Revendications

1. Compositions de dérivés de polyphénols **caractérisées en ce qu'**elles comprennent des oligomères ou des polymères dont les motifs monomères répondent à la formule I. 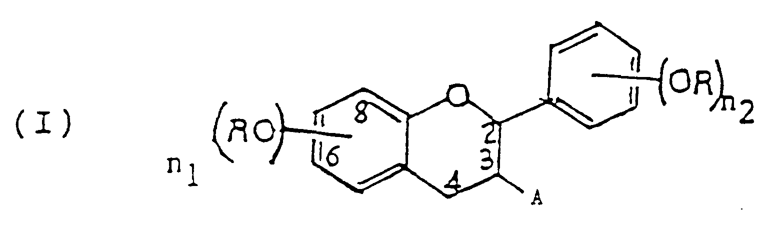 dans laquelle
- A représente un groupe -OR, un atome d'hydrogène, ou un substituant R,
- au moins la majorité des substituants R représente un groupe -COR1, R1 étant un radical alkyle d'au moins deux atomes de carbone, linéaire ou ramifié, saturé ou insaturé, un radical aryle, aralkyle ou aralkylène,
- le ou les autres substituants R qui ne représentent pas un groupe -COR1 étant un atome d'hydrogène, un groupe alkyle, un groupe acyle -CO-C₆H₂-(OH)₃, un ose ou un polyose, et
- n₁ et n₂, identiques ou différents l'un de l'autre, sont des nombres de 1 à 3, correspondant au nombre de substitutions sur un cycle,
et les diastéréoisomères et les régioisomères de ces motifs,
les motifs monomères étant reliés par des liaisons carbone-carbone et/ou par des ponts éthers entre les cycles constitutifs du noyau flavane.

2. Compositions selon la revendication 1, **caractérisées en ce qu'**elles répondent à la formule II 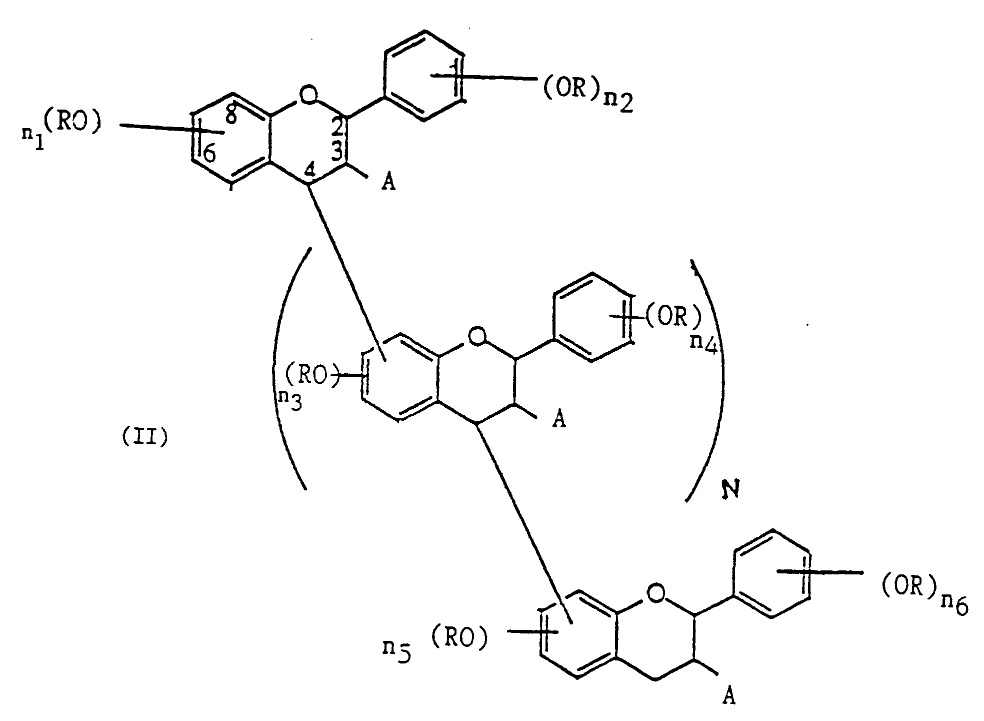 dans laquelle
- A et R sont tels que définis dans la revendication 1,
- n1 à n6, identiques ou différents les uns des autres, sont des nombres de 1 à 3, et
- N est un nombre de 0 à 100,
et les diastéréoisomères et les régioisomères.

3. Compositions selon la revendication 1 ou 2 **caractérisées en ce que** les liaisons entre les atomes de carbone des motifs successifs sont situées entre le C-4 d'un motif et le C-6 ou le C-8 d'un autre motif.

4. Compositions selon l'une des revendications 1 à 3, **caractérisées en ce qu'**au moins deux motifs sont reliés en outre par un pont oxygène.

5. Compositions selon la revendication 4,
**caractérisées en ce que** les oligomères et polymères répondent à la formule III 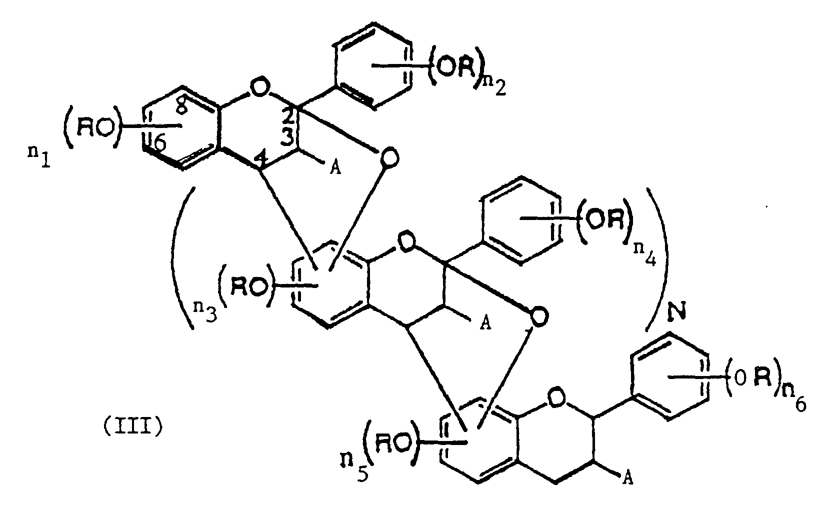 dans laquelle A, R, n₁ à n₆ et N sont tels que définis dans la revendication 2.

6. Compositions selon l'une des revendications 1 à 5, **caractérisées en ce que** N est égal à 0.

7. Compositions selon l'une des revendications 1 à 5, **caractérisées en ce que** N est un nombre de 1 à 10.

8. Compositions selon l'une des revendications 1 à 5, **caractérisées en ce que** N est un nombre de 11 à 100.

9. Compositions selon l'une des revendications 1 à 8, **caractérisées en ce que** R1 représente un radical d'acide gras saturé ou insaturé, en particulier choisi dans le groupe comprenant les radicaux de l'acide butyrique, valérique, hexanique, sorbique, laurique, palmitique, stéarique, oléique, linoléique, linolénique (alpha ou gamma), éicopentanoïque, docosahexaénoïque ou arachidonique.

10. Compositions selon l'une des revendications 1 à 8, **caractérisées en ce que** R1 représente un groupe aryle tel que phényle ou un groupe aralkyle ou aralkylène tel que benzyle ou styryle.

11. Compositions selon l'une des revendications précédentes, **caractérisées en ce qu'**elles renferment en mélange avec les esters oligomères et/ou polymères, majoritairement présents, selon l'une des revendications 1 à 11, des esters monomères selon la revendication 1, des esters des motifs monomères selon la revendication 1.

12. Compositions selon l'une des revendications précédentes, **caractérisées en ce qu'**il s'agit de dérivés de flavanols et qu'ils répondent aux formules I, II ou III dans lesquelles A représente un groupe -OR, où R est tel que défini dans la revendication 1.

13. Compositions selon la revendication 12, **caractérisées en ce que** les groupes oxygénés sont au nombre de 5 par motif flavanolique et occupent les positions 3, 5, 7, 3' et 4', ces esters comportant le cas échéant un pont oxygène entre le C-2 et l'un des carbones C₅ à C₈.

14. Procédé de synthèse de compositions de polyphénols selon la revendication 1, **caractérisé en ce qu'**il comprend la réaction de compositions phénoliques ayant au moins un groupe -OH libre avec un agent d'acylation susceptible de fournir le radical -COR1 tel que défini ci-dessus, dans des conditions permettant la substitution d'au moins un groupe -OH libre par un radical acyle -COR1.

15. Procédé selon la revendication 14, **caractérisé en ce qu'**on utilise comme agent d'acylation un acide R1COOH, ou un dérivé d'un tel acide tel un halogénure, un anhydride ou un ester.

16. Procédé selon la revendication 15, **caractérisé en ce que** lorsqu'on utilise R1COOH comme agent d'acylation, on réalise la réaction en présence d'un agent d'activation de l'acide tel que le dicyclohexylcarbodimide ou le ter-butylchloroformiate.

17. Procédé selon l'une des revendications 14 à 16, **caractérisé en ce que** la réaction est effectuée en présence d'un solvant permettant une solubilisation partielle des composés phénoliques de départ, tels que des dérivés halogénés comme le dichlorométhane, le chloroforme, le 1,2-dichloroéthane, ou une amine comme la pyridine.

18. Procédé selon la revendication 15, **caractérisé en ce que** la réaction avec le chlorure ou l'anhydride d'acide est réalisée en milieu aqueux alcalin, selon la réaction de Schotten Bauman.

19. Préparations pharmaceutiques, **caractérisées en ce qu'**elles renferment une quantité efficace d'au moins une composition selon l'une des revendications 1 à 13, en combinaison avec un véhicule inerte approprié pour une application pharmaceutique.

20. Préparations cosmétiques, **caractérisées en ce qu'**elles renferment au moins une composition selon l'une des revendications 1 à 13, en combinaison avec un véhicule inerte approprié pour une application cosmétique.

21. Application des compositions selon l'une des revendications 1 à 13 en diététique.

## Patentansprüche

1. Zusammensetzung von Polyphenolderivaten, **dadurch gekennzeichnet, dass** sie Oligomere oder Polymere mit Monomereinheiten der Formel I umfassen, 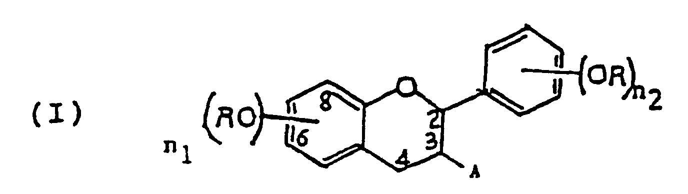 worin
- A für eine Gruppe -OR, ein Wasserstoffatom oder einen Substituenten R steht,
- zumindest der überwiegende Teil der Substituenten R für eine Gruppe -COR¹ stehen, wobei R¹ für einen linearen oder verzweigten, gesättigten oder ungesättigten Alkylrest mit wenigstens zwei Kohlenstoffatomen, einen Aryl-, Aralkyl- oder Aralkylenrest steht,
- der oder die anderen Substituenten R, die nicht für eine Gruppe -COR¹ stehen, für ein Wasserstoffatom, eine Alkylgruppe, eine Acylgruppe -CO-C₆H₂-(OH)₃, eine Ose oder eine Polyose stehen und
- n₁ und n₂ unabhängig voneinander für Zahlen von 1 bis 3 stehen und der Anzahl der Substituenten an einem Ring entsprechen,
und die Diastereoisomeren und Regioisomeren dieser Einheiten, wobei die Monomereinheiten durch Kohlenstoff-Kohlenstoff-Bindungen und/oder durch Etherbrücken zwischen den Ringen, die den Flavan-Kern bilden, verbunden sind.

2. Zusammensetzungen nach Anspruch 1, **dadurch gekennzeichnet, dass** sie der Formel II entsprechen 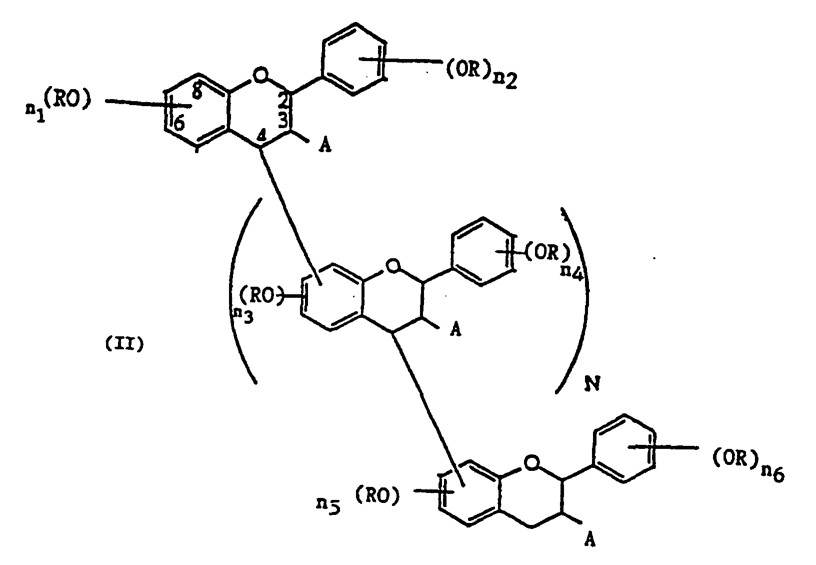 worin
- A und R wie in Anspruch 1 definiert sind,
- n₁ bis n₆ unabhängig voneinander für Zahlen von 1 bis 3 stehen, und
- N für eine Zahl von 0 bis 100 steht,
und die Diastereoisomeren und die Regioisomeren.

3. Zusammensetzungen nach einem der Ansprüche 1 oder 2, **dadurch gekennzeichnet, dass** die Bindungen zwischen den Kohlenstoffatomen der aufeinander folgenden Einheiten das C-4-Atom der einen und das C-6- oder das C-8-Atom der anderen Einheit verbinden.

4. Zusammensetzungen nach einem der Ansprüche 1 bis 3, **dadurch gekennzeichnet, dass** wenigstens zwei Einheiten zusätzlich durch eine Sauerstoffbrücke verbunden sind.

5. Zusammensetzungen nach Anspruch 4, **dadurch gekennzeichnet, dass** die Oligomeren und Polymeren der Formel III entsprechen 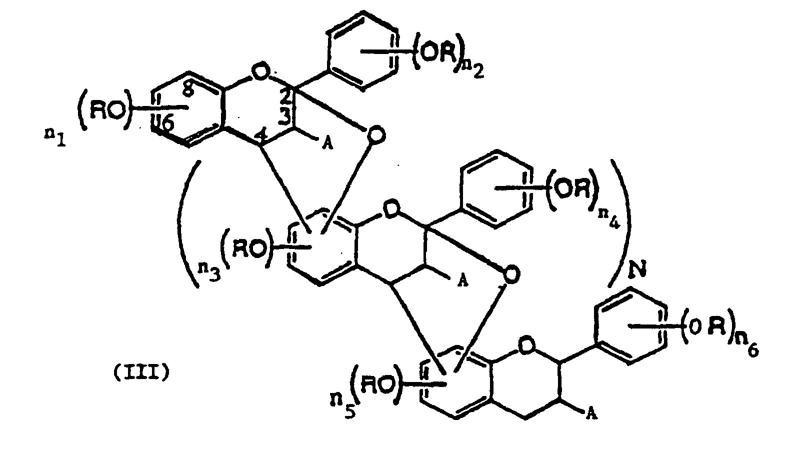 worin A, R, n₁ bis n₆ und N wie in Anspruch 2 definiert sind.

6. Zusammensetzungen nach einem der Ansprüche 1 bis 5, **dadurch gekennzeichnet, dass** N gleich 0 ist.

7. Zusammensetzungen nach einem der Ansprüche 1 bis 5, **dadurch gekennzeichnet, dass** N für eine Zahl von 1 bis 10 steht.

8. Zusammensetzungen nach einem der Ansprüche 1 bis 5, **dadurch gekennzeichnet, dass** N für eine Zahl von 11 bis 100 steht.

9. Zusammensetzungen nach einem der Ansprüche 1 bis 8, **dadurch gekennzeichnet, dass** R¹ für einen gesättigten oder ungesättigten Fettsäurerest steht, insbesondere ausgewählt unter den Resten der Buttersäure, Valeriansäure, Hexansäure, Sorbinsäure, Laurinsäure, Palmitinsäure, Stearinsäure, Ölsäure, Linolsäure, Linolensäure (α oder γ), Eicosapentaensäure, Docosahexaensäure oder Arachidonsäure.

10. Zusammensetzungen nach einem der Ansprüche 1 bis 8, **dadurch gekennzeichnet, dass** R¹ für eine Arylgruppe wie Phenyl oder für eine Aralkyl- oder Aralkylengruppe wie Benzyl oder Styryl steht.

11. Zusammensetzungen nach einem der vorhergehenden Ansprüche, **dadurch gekennzeichnet, dass** sie im Gemisch mit den oligomeren und/oder polymeren Estern nach einem der Ansprüche 1 bis 11, die den überwiegenden Teil bilden, monomere Ester nach Anspruch 1, Ester mit Monomereinheiten nach Anspruch 1 enthalten.

12. Zusammensetzungen nach einem der vorhergehenden Ansprüche, **dadurch gekennzeichnet, dass** es sich um Flavanolderivate handelt und diese den Formeln I, II oder III entsprechen, worin A für eine Gruppe -OR steht, worin R wie in Anspruch 1 definiert ist.

13. Zusammensetzungen nach Anspruch 12, **dadurch gekennzeichnet, dass** jede Flavanoleinheit fünf sauerstoffhaltige Gruppen in den Positionen 3, 5, 7, 3' und 4' aufweist, wobei diese Ester gegebenenfalls eine Sauerstoffbrücke zwischen C-2 und einem der Kohlenstoffatome C-5 bis C-8 aufweisen.

14. Verfahren zur Herstellung von Polyphenolverbindungen nach Anspruch 1, **dadurch gekennzeichnet, dass** es die Umsetzung von Phenolverbindungen, die wenigstens eine freie OH-Gruppe aufweisen, mit einem Acylierungsmittel, das einen -COR¹-Rest, der wie oben definiert ist, übertragen kann, unter Bedingungen umfasst, die die Substitution wenigstens einer freien OH-Gruppe durch ein Acylradikal -COR¹ erlaubt.

15. Verfahren nach Anspruch 14, **dadurch gekennzeichnet, dass** man als Acylierungsmittel eine Säure R¹COOH oder ein Säurederivat, beispielsweise ein Halogenid, Anhydrid oder Ester, verwendet.

16. Verfahren nach Anspruch 15, **dadurch gekennzeichnet, dass** man bei der Verwendung von R¹COOH als Acylierungsmittel die Reaktion in Anwesenheit eines Aktivierungsmittel für Säuren wie Dicyclohexylcarbodiimid oder tert-Butylchloroformiat durchführt.

17. Verfahren nach einem der Ansprüche 14 bis 16, **dadurch gekennzeichnet, dass** die Reaktion in Anwesenheit eines Lösungsmittels, beispielsweise eines Halogenderivats wie Dichlormethan, Chloroform oder 1,2-Dichlorethan oder eines Amids wie Pyridin, durchführt, das die Teilsolubilisierung der Ausgangs-Phenolverbindungen erlaubt.

18. Verfahren nach Anspruch 15, **dadurch gekennzeichnet, dass** die Umsetzung mit dem Säurechlorid oder -anhydrid in wässrigalkalischem Medium nach Schotten-Bauman durchgeführt wird.

19. Pharmazeutische Zusammensetzungen, **dadurch gekennzeichnet, dass** sie eine wirksame Menge wenigstens einer Verbindung nach einem der Ansprüche 1 bis 13 zusammen mit einem inerten Vehikel, das für eine pharmazeutische Anwendung geeignet ist, umfassen.

20. Kosmetische Zusammensetzungen, **dadurch gekennzeichnet, dass** sie wenigstens eine Verbindung nach einem der Ansprüche 1 bis 13 zusammen mit einem inerten Vehikel, das für eine kosmetische Anwendung geeignet ist, umfassen.

21. Verwendung der Verbindungen nach einem der Ansprüche 1 bis 13 in Nährstoffpräparaten.

## Claims

1. Polyphenol derivative compositions, **characterized in that** they comprise oligomers or polymers whose monomer moieties correspond to the formula I. 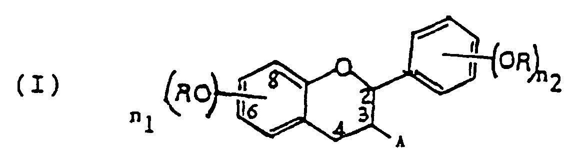 in which
- A represents a group -OR, a hydrogen atom or a substituent R,
- at least the majority of the substituents R represent a group -COR₁, R₁ being a saturated or unsaturated, linear or branched alkyl radical of at least two carbon atoms, or an aryl, aralkyl or aralkylene radical,
- the other substituent or substituents R which do not represent a group -COR₁ being a hydrogen atom, an alkyl group, an acyl group -CO-C₆H₂-(OH)₃, a monosaccharide or a polysaccharide, and
- n₁ and n₂, which are identical to or different from each other, are numbers from 1 to 3, corresponding to the number of substitutions on a ring,
and the diastereoisomers and the regioisomers of these moieties,
the monomer moieties being connected by carbon-carbon bonds or by ether bridges between the rings which make up the flavan ring system.

2. Compositions according to claim 1, **characterized in that** they correspond to the formula II 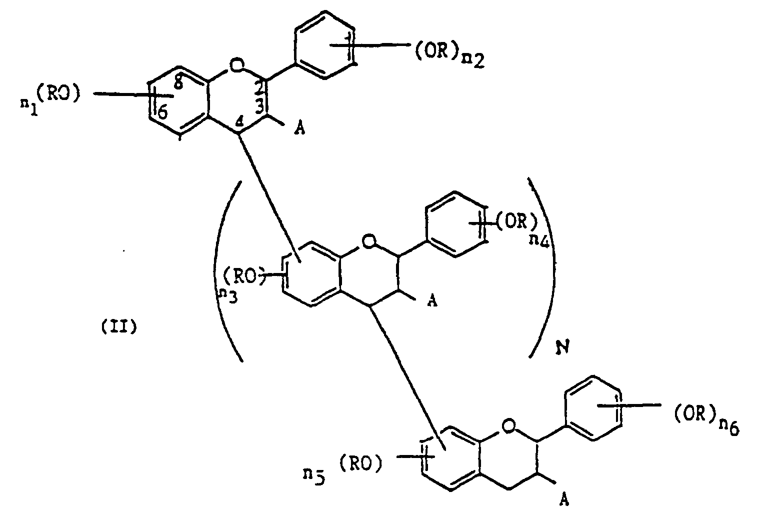 in which
- A and R are as defined in claim 1,
- n₁ to n₆, which may be identical to or different from each other, are numbers from 1 to 3, and
- N is a number from 0 to 100,
and the diastereoisomers and regioisomers.

3. Compositions according to claim 1 or 2, **characterized in that** the bonds between the carbon atoms of the successive moieties are located between C-4 of one moiety and C-6 or C-8 of another moiety.

4. Compositions according to one of claims 1 to 3, **characterized in that** at least two moieties are also connected by an oxygen bridge.

5. Compositions according to claim 4, **characterized in that** the oligomers and polymers correspond to the formula III 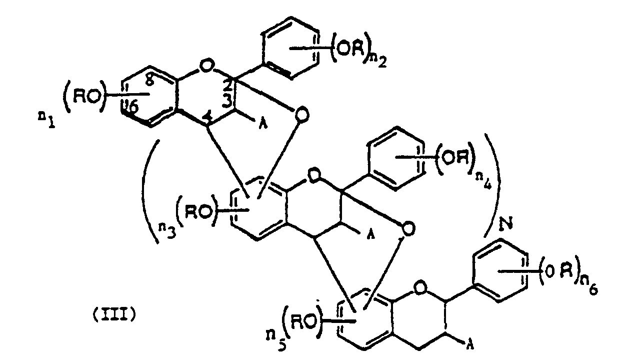 in which A, R, n₁ to n₆ and N are as defined in Claim 2.

6. Compositions according to one of claims 1 to 5, **characterized in that** N is equal to 0.

7. Compositions according to one of claims 1 to 5, **characterized in that** N is a number from 1 to 10.

8. Compositions according to one of claims 1 to 5, **characterized in that** N is a number from 11 to 100.

9. Compositions according to one of claims 1 to 8, **characterized in that** R₁ represents a saturated or unsaturated fatty acid radical chosen in particular from the group comprising butyric, valeric, hexanic [sic], sorbic, lauric, palmitic, stearic, oleic, linoleic, linolenic (alpha or gamma), eicopentanoic [sic], docosahexaenoic or arachidonic acid radicals.

10. Compositions according to one of claims 1 to 8, **characterized in that** R₁ represents an aryl group such as phenyl or an aralkyl or aralkylene group such as benzyl or styryl.

11. Compositions according to one of the preceding claims, **characterized in that** they contain, as a mixture with the oligomer and/or polymer esters mainly present, according to one of claims 1 to 11, monomer esters according to claim 1, esters of monomer moieties according to claim 1.

12. Compositions according to one of the preceding claims, **characterized in that** they are flavanol derivatives and **in that** they correspond to formulae I, II or III in which A represents a group -OR, where R is as defined in claim 1.

13. Compositions according to claim 12, **characterized in that** there are five oxygen-containing groups per flavanol moiety and they occupy positions 3, 5, 7, 3' and 4', these esters containing, where appropriate, an oxygen bridge between C-2 and one of the carbons C₅ to C₈.

14. Process for the synthesis of polyphenol compositions according to claim 1, **characterized in that** it comprises the reaction of phenol compositions having at least one free -OH group with an acylating agent capable of providing the radical -COR₁ as defined above, under conditions which make it possible to substitute at least one free -OH group with an acyl radical -COR₁.

15. Process according to claim 14, **characterized in that** an acid R₁COOH or a derivative of such an acid, such as a halide, an anhydride or an ester, is used as acylating agent.

16. Process according to claim 15, **characterized in that** when R₁COOH is used as acylating agent, the reaction is performed in the presence of an acid-activating agent such as dicyclohexylcarbodiimide or tert-butyl chloroformate.

17. Process according to one of claims 14 to 16, **characterized in that** the reaction is carried out in the presence of a solvent which allows partial solubilization of the starting phenol compounds, such as of the halogenated derivatives such as dichloromethane, chloroform, 1,2-dichloroethane, or an amine such as pyridine.

18. Process according to claim 15, **characterized in that** the reaction with the acid chloride or anhydride is performed in aqueous alkaline medium, according to the Schotten-Bauman [sic] reaction.

19. Pharmaceutical preparations, **characterized in that** they contain an effective amount of at least one composition according to one of Claims 1 to 13, in combination with an inert vehicle which is suitable for a pharmaceutical application.

20. Cosmetic preparations, **characterized in that** they contain at least one composition according to one of claims 1 to 13, in combination with an inert vehicle which is suitable for a cosmetic application.

21. Application of the compositions according to one of claims 1 to 13 in dietetics.
